# EUROPEAN PATENT APPLICATION

(11) **EP 3 361 443 A1**
(43) Date of publication of application: **15.08.2018**
(21) Application number: 16853525.0
(22) Date of filing: 03.10.2016
(51) Int. Cl.: G06Q 50/22, A61B 5/00, A61B 5/11, A61G 12/00, G06T 7/20, G08B 21/02, G08B 25/00, G08B 25/04, H04N 7/18

(54) **ACTION DETECTING SYSTEM, ACTION DETECTING DEVICE, ACTION DETECTING METHOD, AND ACTION DETECTING PROGRAM**

(30) Priority: 06.10.2015 JP 2015198356
(71) Applicant: Konica Minolta, Inc., Tokyo 100-7015 (JP)
(72) Inventor: KURAMOTO, Nozomi, Tokyo 100-7015 (JP)
(74) Representative: Alton, Andrew
(86) International application number: PCT/JP2016/079273
(87) International publication number: WO 2017/061371

(57) **Abstract**

An action detection system that can alleviate false notifications that occur in unexpected situations is provided. An action detection system (300) includes an acquisition unit (150) acquiring an image, a storage unit storing a state transition table (222) for specifying transition relationship between a plurality of types of actions, notification action information (226) defining a notification target action, and a current action (224) of the care-recipient, an action detection unit (255) for specifying a transitionable action to which transition can be made from the current action (224) on the basis of the state transition table (222), and detecting the transitionable action on the basis of the image, a transition control unit (260) for updating the current action (224) to a new action detected, a notification unit (270) for, in a case where the new action is the notification target action, providing notification of a type of the new action, and an update unit (285) for, in a case where a first condition defined in advance indicating that it is not suitable to determine the type of the action of the care-recipient is satisfied, reducing the type of the notification target action and updating the transition relationship of the state transition table (222).

## Description

### Technical Field

The present disclosure relates to an action detection system, an action detection device, an action detection method, and an action detection program capable of discriminating type of action of a person.

### Background Art

There is a technique for determining the action of person from the image. This technique is applied to an action detection system that watches actions of care-recipients who need care such as elderly people and patients. The action detection system detects that the care-recipient has performed a dangerous action involving a fall, etc. (for example, waking up or leaving the bed), and informs the care-giver about this. As a result, the care-giver can rush to the care-recipient and prevent the fall and the like of the care-recipient before it happens.

Regarding such an action detection system, JP 2012-170483 A (Patent Literature 1) discloses a state detection device "capable of detecting the state of a subject with high degree of accuracy with a simple configuration".

### Citation List

### Patent Literature

Patent Literature 1: JP 2012-170483 A

### Summary of Invention

### Technical Problem

When an unexpected situation occurs in the action detection system, such as when the camera receives disturbance such as light or when a system failure occurs, the action detection system falsely detects the action of care-recipient. In this case, the number of notifications to the care-giver increases and the burden on the care-giver increases. Therefore, an action detection system capable of suppressing an increase in the burden on the care-giver even when an unexpected situation arises is desired.

The state detection device disclosed in Patent Literature 1 determines the current state of the subject based on the moving direction of the subject on the bed and the past state of the subject. The accuracy of the determination of the state of the target person is improved by considering the movement direction of the target person. However, Patent Literature 1 does not disclose the determination process when an unexpected situation occurs. Therefore, when an unexpected situation occurs in the state detection device, the erroneous determination of the state of the subject increases.

The present disclosure has been made to solve the above-mentioned problems, and it is an object in one aspect to provide an action detection system capable of alleviating false alarm occurring in an unexpected situation. It is an object in another aspect to provide an action detection device capable of alleviating false alarm occurring in an unexpected situation. It is an object in still another aspect to provide an action detection method capable of alleviating false alarm occurring in an unexpected situation. It is an object in still another aspect to provide an action detection program capable of alleviating false alarm occurring in an unexpected situations.

### Solution to Problem

According to one aspect, an action detection system includes: an acquisition unit for acquiring operation data indicating an operation of a subject; a storage unit for storing a state transition table for specifying transition relationship between a plurality of types of actions, a type of a notification target action, and a type of a current action of the subject; an action detection unit for specifying a transitionable action to which transition can be made from the current action on the basis of the state transition table, and detecting the transitionable action on the basis of the operation data; a transition control unit for updating the current action to the new action in a case where the action detection unit detects the new action; a notification unit for, in a case where the new action is detected and the new action is the notification target action, providing notification of a type of the new action; and an update unit for, in a case where a first condition defined in advance indicating that it is not suitable to determine the type of the action of the subject is satisfied, reducing the type of the notification target action and updating the transition relationship of the state transition table.

According to another aspect, an action detection system includes an acquisition unit for acquiring operation data indicating an operation of a subject, a storage unit for storing a state transition table for specifying transition relationship between a plurality of types of actions, and a type of a current action of the subject, an action detection unit for specifying a transitionable action to which transition can be made from the current action on the basis of the state transition table, and detecting the transitionable action on the basis of the operation data, a transition control unit for updating the current action to a new action in a case where the action detection unit detects the new action, and a notification unit for, in a case where the new action is detected and the new action is a notification target action defined in advance, providing notification of a type of the new action, wherein in a case where a condition defined in advance indicating that it is not suitable to determine the type of the action of the subject is satisfied, the notification unit stops providing notification of the notification target action defined in advance.

According to still another aspect, an action detection device includes: an acquisition unit for acquiring operation data indicating an operation of a subject; a storage unit for storing a state transition table for specifying transition relationship between a plurality of types of actions, a type of a notification target action, and a type of a current action of the subject; an action detection unit for specifying a transitionable action to which transition can be made from the current action on the basis of the state transition table, and detecting the transitionable action on the basis of the operation data; a transition control unit for updating the current action to a new action in a case where the action detection unit detects the new action; a notification unit for, in a case where the new action is detected and the new action is the notification target action, providing notification of a type of the new action; an update unit for, in a case where a first condition defined in advance indicating that it is not suitable to determine the type of the action of the subject is satisfied, reducing the type of the notification target action and updating the transition relationship of the state transition table.

According to still another aspect, an action detection method includes: a step of acquiring operation data indicating an operation of a subject; a step of preparing a state transition table for specifying transition relationship between a plurality of types of actions, a type of a notification target action, and a type of a current action of the subject; a step of specifying a transitionable action to which transition can be made from the current action on the basis of the state transition table, and detecting the transitionable action on the basis of the operation data; a step of updating the current action to a new action in a case where the new action is detected in the detecting step; a step of, in a case where the new action is detected and the new action is the notification target action, providing notification of a type of the new action; and a step of, in a case where a first condition defined in advance indicating that it is not suitable to determine the type of the action of the subject is satisfied, reducing the type of the notification target action and updating the transition relationship of the state transition table.

According to still another aspect, an action detection program causes a computer to execute: a step of acquiring operation data indicating an operation of a subject; a step of preparing a state transition table for specifying transition relationship between a plurality of types of actions, a type of a notification target action, and a type of a current action of the subject; a step of specifying a transitionable action to which transition can be made from the current action on the basis of the state transition table, and detecting the transitionable action on the basis of the operation data; a step of updating the current action to a new action in a case where the new action is detected in the detecting step; a step of, in a case where the new action is detected and the new action is the notification target action, providing notification of a type of the new action; and a step of, in a case where a first condition defined in advance indicating that it is not suitable to determine the type of the action of the subject is satisfied, reducing the type of the notification target action and updating the transition relationship of the state transition table.

### Advantageous Effects of Invention

In one aspect, false notification occurring in an unexpected situation can be alleviated.

These and other objects, features, aspects and advantages of the present invention will be understood from the following detailed description of the invention, taken in conjunction with the accompanying drawings.

### Brief Description of Drawings

Fig. 1 is a diagram showing an example of a configuration of an action detection system according to a first embodiment.
Fig. 2 is a diagram for explaining action detection processing by the action detection system according to the first embodiment.
Fig. 3 shows content of a state transition table according to the first embodiment.
Fig. 4 shows an example of action of a care-recipient.
Fig. 5 shows definition of each action state prescribed in the state transition table according to the first embodiment.
Fig. 6 is a conceptual diagram schematically showing action determination processing according to a modification.
Fig. 7 shows transition condition from each action state prescribed in the state transition table according to the first embodiment.
Fig. 8 shows content of the notification action information according to the first embodiment.
Fig. 9 is a diagram showing an example of an image obtained by shooting a room of a care-recipient.
Fig. 10 shows the care-giver caring for the care-recipient.
Fig. 11 is a diagram showing a state transition example in a case where the abnormality condition in the first embodiment is no longer satisfied.
Fig. 12 is a flowchart showing processing of the action detection system when a normality condition is satisfied.
Fig. 13 is a flowchart showing processing of the action detection system when an invalid condition is satisfied.
Fig. 14 is a block diagram showing a main hardware configuration of the action detection device according to the first embodiment.
Fig. 15 shows content of a state transition table according to a second embodiment.
Fig. 16 shows the definition of each action state prescribed in the state transition table according to the second embodiment.
Fig. 17 shows transition condition from each action state prescribed in the state transition table according to the second embodiment.

### Description of Embodiments

Hereinafter, embodiments according to the present invention will be described with reference to the drawings. In the following description, the same parts and configuration elements are denoted with the same reference numerals, and their names and functions are also the same. Therefore, detailed description thereof will not be repeated. It should be noted that each embodiment and each modification described below may be selectively combined as appropriate.

### <First embodiment>

### [Device configuration of action detection system 300]

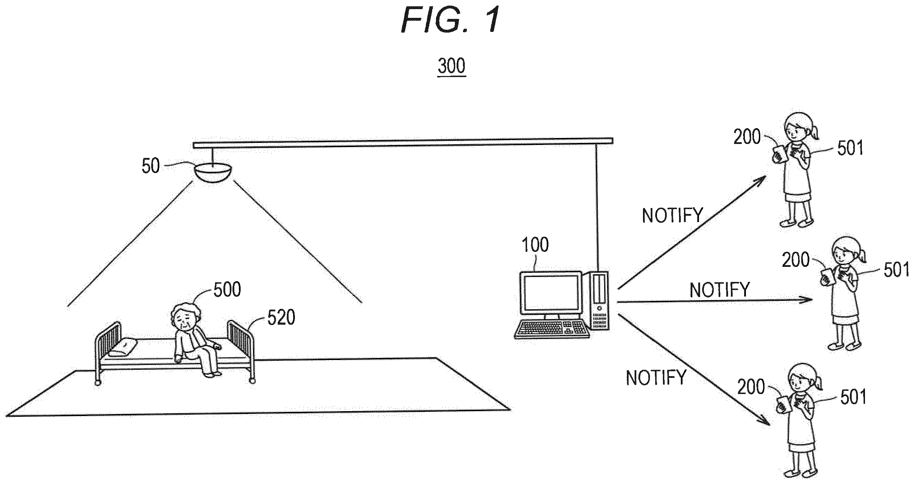

A device configuration of an action detection system 300 will be described with reference to Fig. 1. Fig. 1 shows an example of the configuration of the action detection system 300.

The action detection system 300 can be used, for example, to watch the care-recipient 500 which is a person to be monitored. As shown in Fig. 1, the action detection system 300 includes a camera 50, an action detection device 100, and a mobile terminal 200. The camera 50 and the action detection device 100 are connected to each other via a network. The action detection device 100 and the mobile terminal 200 are mutually connected via a network.

The camera 50 is installed in, for example, a nursing facility, a medical facility, a house, or the like. Fig. 1 shows the camera 50 shooting the care-recipient 500 and a bed 520 from the ceiling. The camera 50 may be attached to the ceiling or attached to the side wall.

The action detection device 100 determines the action of the care-recipient 500 based on the time-series image (i.e., image) obtained from the camera 50. As an example, the action of the care-recipient 500 which the action detection device 100 can detect includes rising of the care-recipient 500, falling off from the bed 520, and the like.

When the action detection device 100 detects the action as the notification target, the action detection device 100 transmits information indicating the type of the action to the mobile terminal 200 for the care-giver 501. When the mobile terminal 200 receives from the action detection device 100 the information indicating the type of the action as the notification target, the mobile terminal 200 notifies the care-giver 501 that an action targeted for notification has been detected. As an example of the notification method, the mobile terminal 200 displays information indicating the type of action as a message, or speaks the type of action in voice. As a result, the care-giver 501 can understand an action such as getting up and falling of the care-recipient 500, and can quickly rush to the care-recipient 500.

Fig. 1 shows an example in which the action detection system 300 includes one camera 50, but the action detection system 300 may include a plurality of cameras 50. In addition, Fig. 1 shows an example in which the action detection system 300 includes one action detection device 100, but the action detection system 300 may include a plurality of action detection devices 100. Furthermore, in Fig. 1, the camera 50 and the action detection device 100 are configured as separate devices, but the camera 50 and the action detection device 100 may be integrally configured. Furthermore, Fig. 1 shows an example in which the action detection system 300 includes a plurality of mobile terminals 200, but the action detection system 300 may be configured with one mobile terminal 200.

### [Action determination method of action detection system 300]

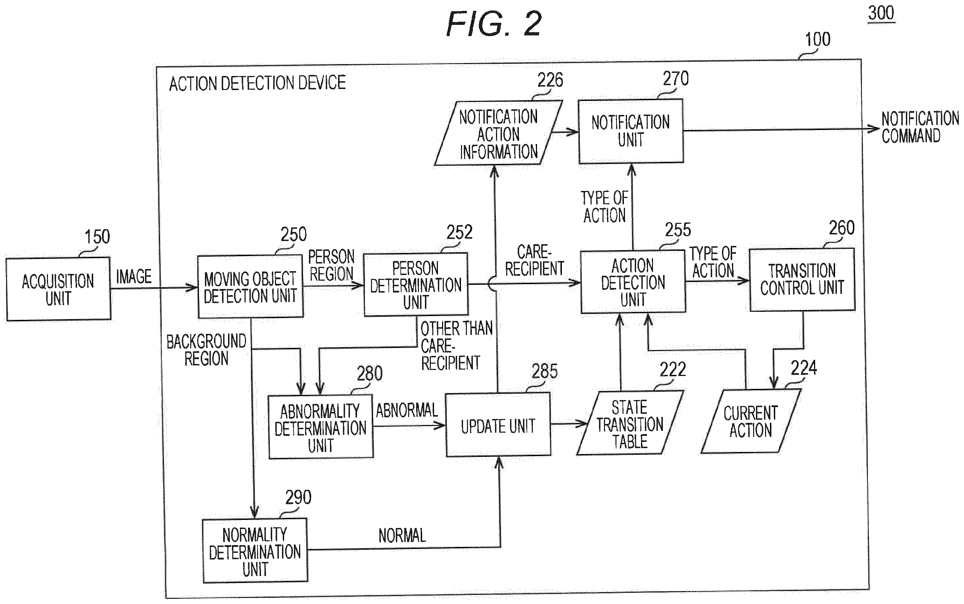

An action determination method by the action detection system 300 will be explained with reference to Fig. 2 to Fig. 11. Fig. 2 is a diagram for explaining action detection processing by the action detection system 300.

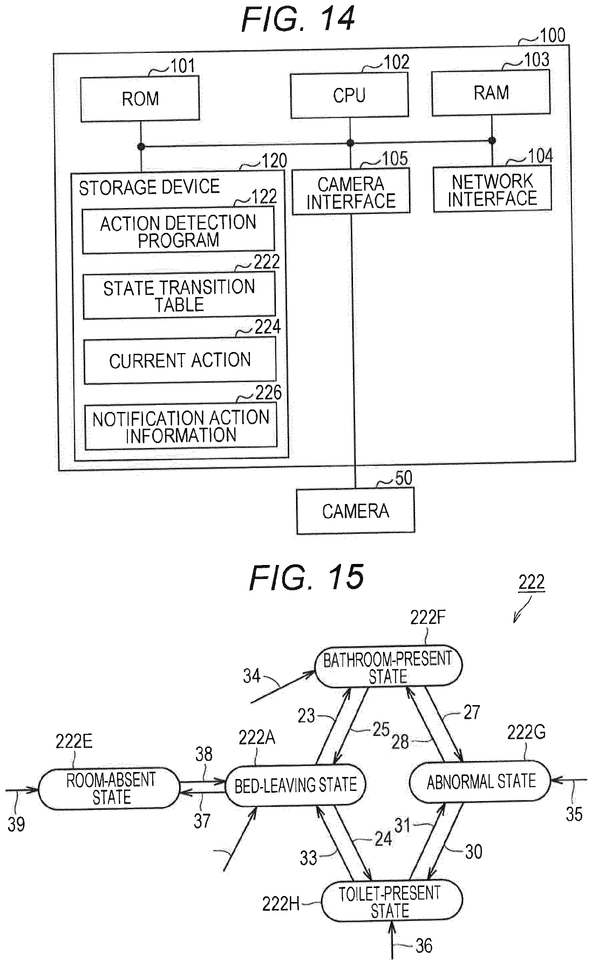

As shown in Fig. 2, the action detection system 300 includes an action detection device 100 and an acquisition unit 150. The action detection device 100 includes, as a function configuration, a moving object detection unit 250, a person determination unit 252, an action detection unit 255, a transition control unit 260, a notification unit 270, an abnormality determination unit 280, an update unit 285, and a normality determination unit 290. The action detection device 100 stores, as data, a state transition table 222, a current action 224 indicating the type of the current action of the care-recipient, and a notification action information 226 defining the type of the notification target action. The state transition table 222, the current action 224, and the notification action information 226 are stored in a storage device 120 (see FIG. 14) etc. of the action detection device 100

In the following description, the acquisition unit 150, the moving object detection unit 250, the person determination unit 252, the action detection unit 255, the transition control unit 260, the notification unit 270, the abnormality determination unit 280, the update unit 285, and the normality determination unit 290 will be sequentially described.

### (Acquisition unit 150)

The acquisition unit 150 acquires operation data representing the operation of a care-recipient. As an example, the acquisition unit 150 is the camera 50 shown in Fig. 1. The acquisition unit 150 generates images obtained by shooting the care-recipient as operation data and outputs the images to the moving object detection unit 250 in chronological order.

In the following, an example will be described in which the acquisition unit 150 is the camera 50 and the operation data is a time-series image (that is, image), but the acquisition unit 150 is not limited to the camera 50, and the operation data is limited to the image. The acquisition unit 150 may be a device capable of acquiring data indicating a temporal change in operation of the care-recipient. For example, the acquisition unit 150 may be a pressure sensitive mat located under the bed, a time-of-flight (TOF) distance sensor, an acceleration sensor, a microwave sensor, or the like. The pressure sensitive mat outputs the pressure value as operation data. The distance sensor outputs distance images as operation data. The acceleration sensor outputs acceleration as operation data. The microwave sensor outputs the distance from itself to the care-recipient as operation data.

### (Moving object detection unit 250)

The moving object detection unit 250 detects a moving object from a time series image obtained from the acquisition unit 150. As an example, a moving object region in an image is realized by a background difference that differentiates a predetermined background image from an input image, or a time difference that differentiates images taken at different shooting times.

The moving object detection unit 250 outputs the moving object region in the image to the person determination unit 252 and outputs the background region other than the moving object region to the abnormality determination unit 280.

### (Person determination unit 252)

The person determination unit 252 determines whether or not the detected moving object region represents a person. In one aspect, the person determination unit 252 determines that the moving object region represents a person when the size of the moving object region is within a predetermined range. In another aspect, the person determination unit 252 determines that the moving object region represents a person when the aspect ratio of the moving object region is within a predetermined range. In yet another aspect, the person determination unit 252 executes face detection in the moving object region, and determines that the moving object region represents a person when a face is detected.

Next, the person determination unit 252 determines whether or not the detected person region represents a previously registered care-recipient. As an example, in the action detection system 300, a feature amount is extracted from an image obtained by shooting the care-recipient, and the feature amount is held in advance as a template. The person determination unit 252 calculates the degree of similarity between the template and the feature amount extracted from the detected person region. When the degree of similarity is equal to or greater than the predetermined value, the person determination unit 252 determines that the detected person region represents the previously registered care-recipient.

If it is known beforehand that a person other than the care-recipient does not exist in the room to be monitored, or when the monitored region is limited to the bed region, the person determination unit 252 does not need to determine the person.

### (Action detection unit 255)

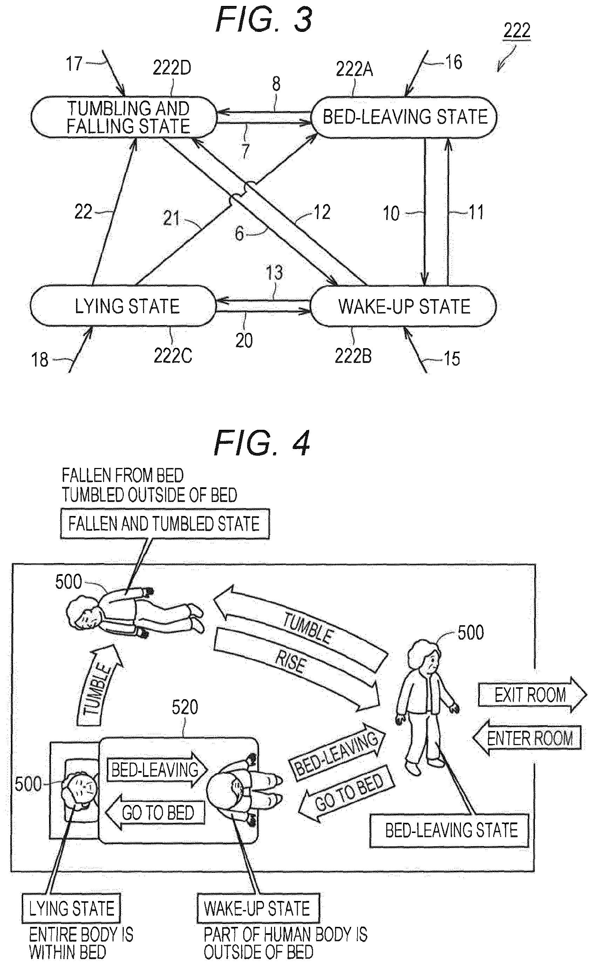

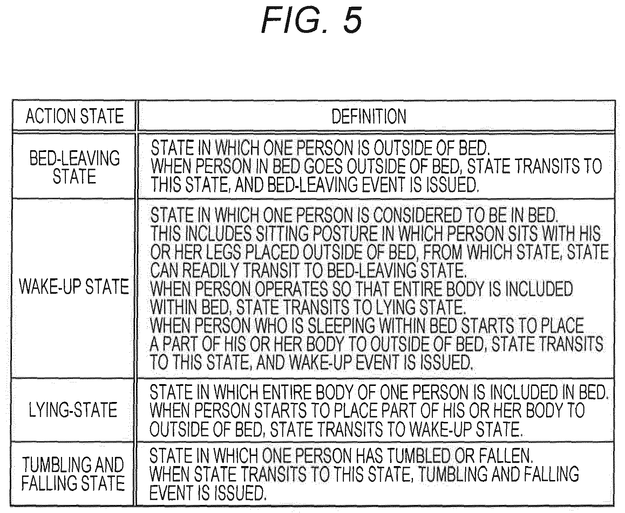

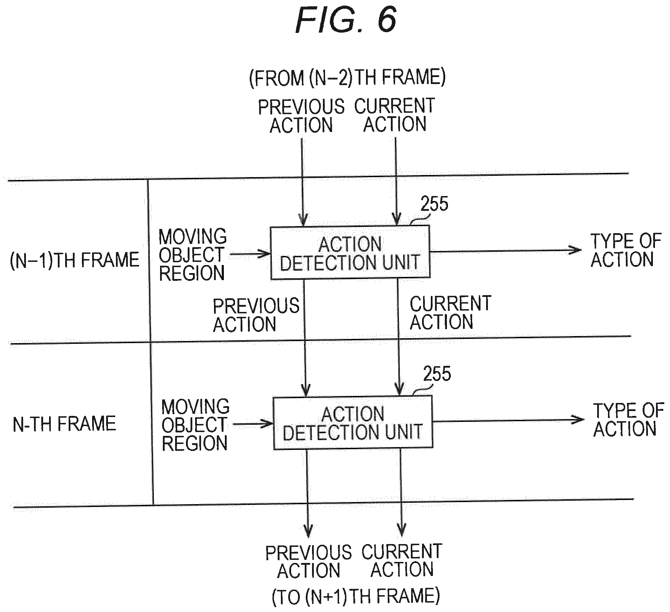

The action determination processing by action detection unit 255 will be explained with reference to Fig. 3 to Fig. 6. Fig. 3 shows content of the state transition table 222. Fig. 4 shows an example of the action of care-recipient 500. Fig. 5 shows the definition of each action state prescribed in the state transition table 222. Fig. 6 is a conceptual diagram schematically showing action determination processing according to modification.

Based on the state transition table 222, the action detection unit 255 specifies a transitionable action from the current action 224, and detects the transitionable action based on the operation data. More specifically, the action detection unit 255 sets an action that can be transitioned from the current action 224 as a determination target action.

In the state transition table 222, a transition relationship between plural types of actions is defined. In the example of Fig. 3, the state transition table 222 defines a bed-leaving state 222A, a wake-up state 222B, a lying state 222C, and a tumbling and falling state 222D as the action state. Fig. 4 shows an example of the action state of the care-recipient 500. Fig. 5 shows the definitions of the bed-leaving state 222A, the wake-up state 222B, the lying state 222C, and the tumbling and falling state 222D

As shown in Fig. 3 and Fig. 4, the action state can transit from the bed-leaving state 222A to the wake-up state 222B or the tumbling and falling state 222D. The action state can transit from the wake-up state 222B to the bed-leaving state 222A, the lying state 222C, or the tumbling and falling state 222D. From the lying state 222C, the action state can transit to the bed-leaving state 222A, the wake-up state 222B, or the tumbling and falling state 222D. From the tumbling and falling state 222D, the action state can transit to bed-leaving state 222A or wake-up state 222B.

Based on the state transition table 222, the action detection unit 255 specifies the type of action that can be transitioned from the current action 224, and detects the transitionable action specified based on the image obtained from the acquisition unit 150. The actions that can be determined by the action detection unit 255 include at least one of wake-up of the care-recipient, bed-leaving of the care-recipient, lying of the care-recipient, and tumbling and falling of the care-recipient, for example.

For example, the action detection unit 255 may determine the type of care-recipient's action based on at least the position of the care-recipient in the image, information about the operation of the care-recipient (e.g., the movement direction or the movement amount within a predetermined time), and the posture of the care-recipient.

A more specific action detection method will be described. The bed region is preset in the image. The bed region may be preset manually or may be automatically detected by an image processing technique such as template matching. When a part of the care-recipient in the image overlaps the bed region, the action detection unit 255 detects wake-up of the care-recipient. When the position of care-recipient in the image is not included in the bed region, the action detection unit 255 detects bed-leaving of care-recipient. When the position of the care-recipient in the image is included in the bed region and the movement amount is equal to or less than a predetermined amount, the action detection unit 255 detects lying of the care-recipient. When the position of the care-recipient in the image is not included in the bed region and the movement amount is equal to or more than a predetermined amount, the action detection unit 255 detects tumbling and falling of the care-recipient.

As a specific example of action detection, it is assumed that the current action 224 indicates the bed-leaving state 222A. In this case, the action detection unit 255 refers to the state transition table 222, and sets, as the detection target action, the wake-up state 222B and the tumbling and falling state 222D that can be transitioned from the bed-leaving state 222A. The action detection unit 255 detects wake-up or tumbling and falling of the subject based on the image obtained from the acquisition unit 150.

In the above example, the action detection unit 255 specifies the determination target action based on the current action 224. As shown in Fig. 6, the action detection unit 255 may further use the previous action to specify the determination target action. That is, based on the state transition table 222, the action detection unit 255 specifies an action to which transition can be made from the previous action and the current action 224, and sets the action as the determination target action.

As an example, in the image of the (N-1)th frame, the action detection unit 255 sets, based on the state transition table 222, the action to which transition can be made from the action detected in the image of the (N-3)th frame and the action detected in the image of the (N-2)th frame as the determination target. Based on the image information of the detected moving object region, the action detection unit 255 determines whether or not a determination target action has occurred, and outputs the type of the detected action to the notification unit 270. The action detection unit 255 sets the action detected in the image of the (N-1)th frame as the current action 224, and sets the action detected in the image of the (N-2)th frame as the previous action, so that the action detection unit 255 prepares for action detection in the image of the N-th frame subsequent thereto.

Similarly, in the image of the N-th frame, the action detection unit 255 sets, based on the state transition table 222, the action to which transition can be made from the previous action and the current action 224 as the determination target. Based on the image information of the detected moving object region, the action detection unit 255 determines whether or not a determination target action has occurred, and outputs the type of the detected action to the notification unit 270. The action detection unit 255 sets the action detected in the image of the N-th frame as the current action 224, and sets the action detected in the image of the (N-1)th frame as the previous action, so that the action detection unit 255 prepares for action detection in the image of the (N+1)th frame subsequent thereto.

### (Transition control unit 260)

When a new action is detected by the action detection unit 255, the transition control unit 260 updates the current action 224 with the new action. As a result, the transition control unit 260 causes the current action 224 to transit to the next state.

As the update method of the current action 224, the transition control unit 260 may rewrite the current action 224 with the newly detected action, or may sequentially add the newly detected action as history information to the current action 224.

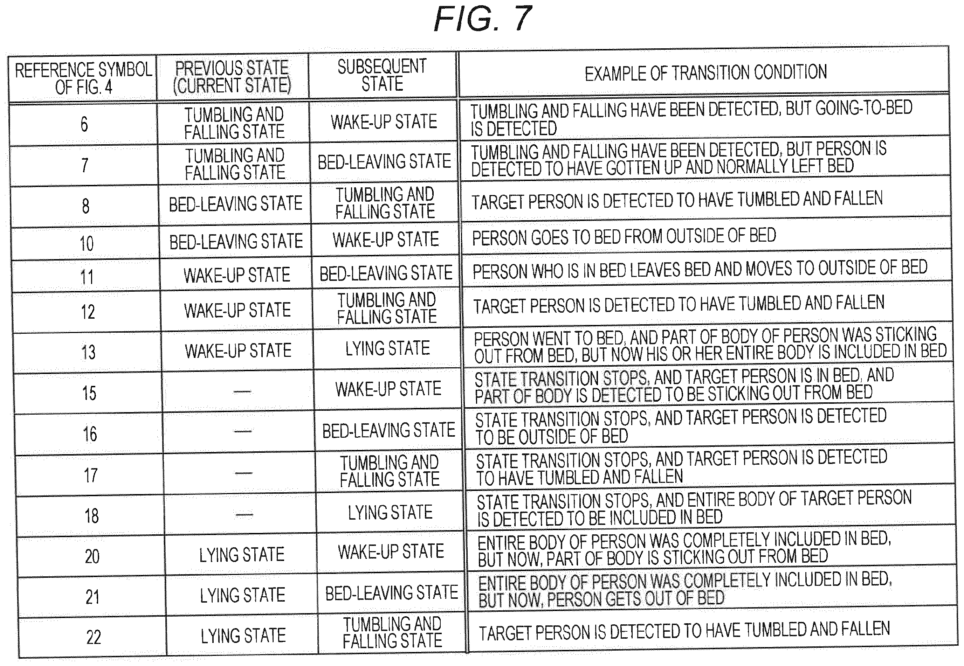

Fig. 7 shows transition condition from each action state prescribed in the state transition table 222. More specifically, a transition condition 6 indicates a transition condition from tumbling and falling state 222D to wake-up state 222B. A transition condition 7 indicates a transition condition from the tumbling and falling state 222D to the bed-leaving state 222A. A transition condition 8 indicates a transition condition from the bed-leaving state 222A to the tumbling and falling state 222D. A transition condition 10 indicates a transition condition from the bed-leaving state 222A to the wake-up state 222B. A transition condition 11 indicates a transition condition from the wake-up state 222B to the bed-leaving state 222A. A transition condition 12 indicates a transition condition from the wake-up state 222B to the tumbling and falling state 222D. A transition condition 13 indicates a transition condition from the wake-up state 222B to the lying state 222C. A transition condition 15 indicates a transition condition from the initial state where the action state is not determined to the wake-up state 222B. A transition condition 16 indicates a transition condition from the initial state to the bed-leaving state 222A. A transition condition 17 indicates a transition condition from the initial state to the tumbling and falling state 222D. A transition condition 18 indicates a transition condition from the initial state to the lying state 222C. A transition condition 20 indicates a transition condition from the lying state 222C to the wake-up state 222B. A transition condition 21 indicates a transition condition from the lying state 222C to the bed-leaving state 222A. A transition condition 22 indicates a transition condition from the lying state 222C to the tumbling and falling state 222D.

In the transition condition shown in Fig. 7, other conditions may be prescribed. For example, when a vibration sensor is used for action detection instead of a camera, it is determined whether or not the transition conditions 10 and 11 are satisfied according to the output from the vibration sensor. Besides, when the temperature sensor and the distance sensor are used for action detection, it is determined whether or not the transition condition 20 is satisfied according to the outputs from the temperature sensor and the distance sensor.

### (Notification unit 270)

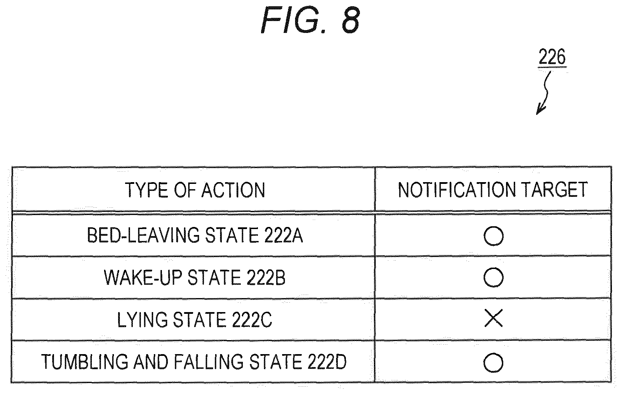

The notification processing by notification unit 270 will be described with reference to Fig. 8. Fig. 8 shows content of the notification action information 226.

The notification unit 270 provides notification of the type of the new action when a new action is detected by the action detection unit 255 and the new action is a notification target action. Whether or not a notification target is set is prescribed in the notification action information 226 in advance, for example.

As shown in Fig. 8, the notification action information 226 indicates whether or not notification targets are set in accordance with the types of actions. In the example of Fig. 8, the bed-leaving state 222A, the wake-up state 222B, and the tumbling and falling state 222D are prescribed as the notification targets. The lying state 222C is prescribed as a non-notification target.

When a new action is detected by the action detection unit 255, the notification action information 226 is referred to. If the notification action information 226 prescribes that the new action is a notification target, the notification action information 226 notifies the care-giver of the content of the new action.

As an example of the notification method, the notification unit 270 displays information indicating the type of the action as a message on the mobile terminal 200 (see Fig. 1) of a care-giver. Alternatively, the notification unit 270 causes the mobile terminal 200 of the care-giver to output the type of the action by voice.

The type of the action that can be the notification target includes at least one of wake-up of the care-recipient, bed-leaving of the care-recipient, fall of the care-recipient, tumbling of the care-recipient, going-to-bed of the care-recipient, lying of the care-recipient, room-entering of the care-recipient, and the room-exiting of the care-recipient. As an example, the target where the care-recipient performs room-entering includes at least one of a care-recipient's room, a toilet, and a bath. The target where the care-recipient performs room-exiting includes at least one of the care-recipient's room, the toilet, and the bath.

The notification target action may be arbitrarily set by the administrator of the action detection system 300. More preferably, the notification target action is set for each transition condition shown in Fig. 7. The administrator can make setting, for example, so as to provide notification of wake-up when the transition condition 20 is satisfied, and not to provide notification of wake-up when the transition condition 10 is satisfied.

The notification of the target action is not necessarily performed when the transition condition is satisfied. For example, when the invalid condition is satisfied, the notification unit 270 may stop providing notification of the predetermined notification target action, and in this case, even when the transition condition is satisfied, the notification of the target action is not performed.

### (Abnormality determination unit 280)

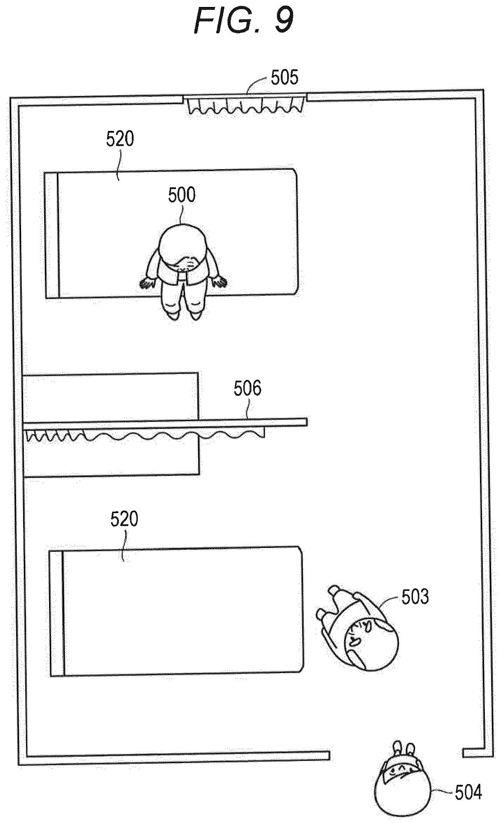

The abnormality determination processing by the abnormality determination unit 280 will be explained with reference to Fig. 9. Fig. 9 shows an example of an image obtained by shooting a room of a care-recipient 500.

When an unexpected situation occurs, e.g., such as a case where the camera is disturbed by light and the like or a case where the action detection system 300 is malfunctioning, there is a high possibility that the action of the care-recipient 500 will be falsely detected. The abnormality determination unit 280 determines whether a predetermined condition is satisfied or not. The predetermined condition indicates that it is not suitable to determine the type of the action of the care-recipient 500 (hereinafter referred to as "invalid condition") in order to determine whether or not an unexpected situation has occurred. When the invalid condition is satisfied, the update unit 285 updates the state transition table 222 (see Fig. 3) and the notification action information 226 (see Fig. 8), as will be described later, to reduce the notification target action types. This alleviates false notifications that occur in unexpected situations.

An example of an unexpected situation includes, as shown in Fig. 9, a case where an obstacle such as a curtain 505 or a partition 506 appearing in the image is moved. Besides, there are cases where the camera is affected by light, such as when the lighting of the room is turned on or turned off. These disturbances are falsely detected as moving objects, and can be causes of falsely detecting the action of care-recipient 500. Therefore, the invalid condition is satisfied when disturbance occurs in the image.

When a disturbance is occurring, there is a great change in pixel values in a region including the background other than the care-recipient in the image (hereinafter also referred to as "background region"). Therefore, the abnormality determination unit 280 uses image information in the background region to determine whether or not disturbance has occurred. As an example, when the change amount of the pixel value in the background region is larger than a predetermined amount, the abnormality determination unit 280 determines that disturbance is occurring in the image, and determines that the invalid condition is satisfied.

Another example of an unexpected situation is a case where there is another person (e.g., persons 503 and 504) such as a care-giver in the room of the care-recipient 500. When another person is present in the room, it is unlikely that the action of the care-recipient 500 is monitored. Therefore, the abnormality determination unit 280 determines that the invalid condition is satisfied when a person other than the care-recipient 500 is detected in the image by the person determination unit 252 (see Fig. 2). Whether the invalid condition is satisfied or not may be determined on the basis of whether or not a person other than the care-recipient 500 is included in the image as described above, or, may be determined on the basis of the image information in the background region as described above.

Another example of an unexpected situation is a case where the action detection system 300 is initialized (reset) by an administrator. Other than the above, there may be a case where a network failure or other error occurs in the action detection system 300. In these cases, the current action 224 may not be acquired and the action detection system 300 may not be able to accurately determine the care-recipient's action. Therefore, the abnormality determination unit 280 determines that the invalid condition is satisfied when the action detection system 300 is initialized, or when an error occurs in the action detection system 300.

As described above, the invalid condition is satisfied in at least one of the following cases: a case where disturbance is generated in the image, a case where a person other than the care-recipient 500 is included in the image, a case where the action detection system 300 is initialized, and a case where an error has occurred in the action detection system 300.

### (Update unit 285)

When the invalid condition is satisfied, the update unit 285 reduces the types of the notification target actions prescribed in the notification action information 226 (see Fig. 8) than in the normal state, and also updates the transition relationship in the state transition table 222 (see Fig. 3). As an example, the update unit 285 updates the state transition table 222 so as not to transition to at least some of the notification target actions when the invalid condition is satisfied. The state transition table after the update is held in the storage device of the action detection system 300 in advance, for example. When the invalid condition is satisfied, the type of the notification target action is decreased, so that false notification to the care-giver is suppressed even if false detection occurs. As a result, the burden on the care-giver is reduced.

More preferably, the update unit 285 sets the type of the notification target action prescribed in the notification action information 226 to zero. As a result, the action detection system 300 can reliably prevent a false notification occurring when an unexpected situation occurs. While the notification is stopped, the state transition in the state transition table 222 may be executed.

The type of action to which transition can be made from the current action 224 (see Fig. 2) is specified based on the updated state transition table, while the abnormality condition is satisfied. As a result, the notification target action is less likely to be detected, which suppresses false notification.

As the update method of the state transition table 222, the update unit 285 may rewrite the state transition table 222 according to update information prepared in advance in preparation for an unexpected situation, or may replace the state transition table 222 with a state transition table prepared in advance in preparation for an unexpected situation.

When the invalid condition is satisfied, the transition itself in the state transition table 222 may be stopped. As a result, the notification target action is never detected, so that false notification is suppressed.

### (Normality determination unit 290)

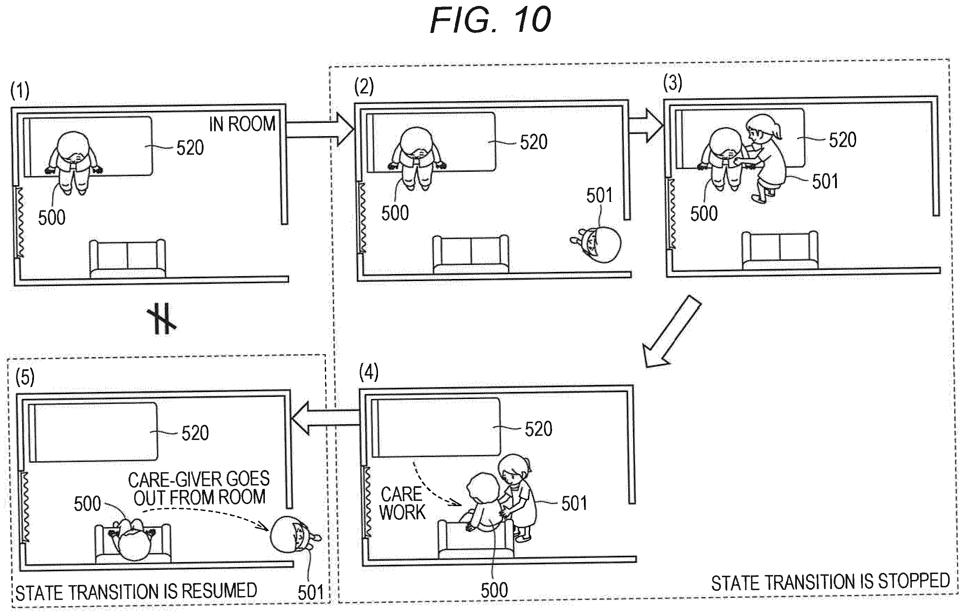

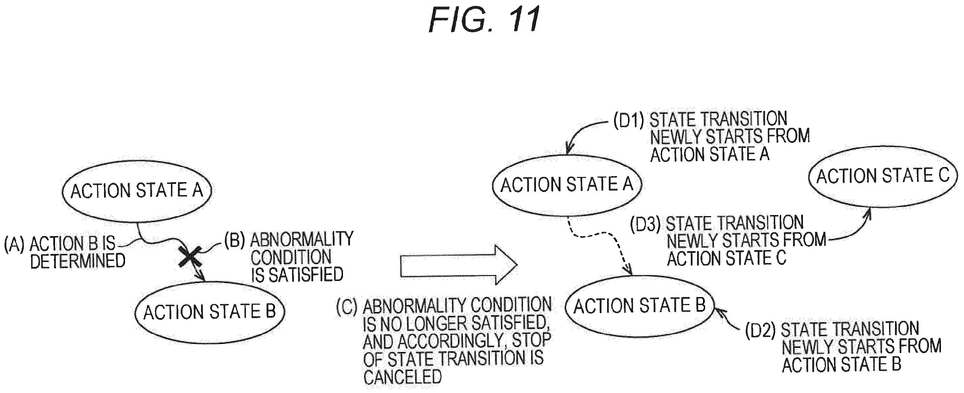

The normal determination processing by the normality determination unit 290 will be explained with reference to Fig. 10 and Fig. 11. Fig. 10 shows the care-giver 501 caring for the care-recipient 500. Fig. 11 is a diagram showing an example of state transition when the abnormality condition is no longer satisfied.

As described above, when the invalid condition indicating that it is not suitable to determine the type of the care-recipient's action is satisfied, the notification to the care-giver is suppressed. Thereafter, the normality determination unit 290 determines whether or not the invalid condition has been resolved. That is, the normality determination unit 290 determines whether or not a normality condition indicating that it is suitable to determine the type of the care-recipient action is satisfied. When the normality condition is satisfied, the update unit 285 returns the state transition table 222 which has been updated so as to suppress the notification back to the original state transition table 222, and also returns the type of the notification target action back to the original state .

The normality condition may be a condition opposite to the invalid condition or a condition not related to the invalid condition. In a case where the normality condition is a condition opposite to the invalid condition, the normality condition is satisfied when the invalid condition is not satisfied.

As described above, when the invalid condition is satisfied, the action detection system 300 may perform control to stop the transition in the state transition table 222. When the normality condition is satisfied and the state transition is resumed, the action state of the care-recipient 500 may be different between before the state transition is stopped and after the state transition is resumed. An example of this is shown in Fig. 10.

In step (1), only the care-recipient 500 is present in the room. In step (1), the normality condition is satisfied.

In step (2), it is assumed that the care-giver 501 has entered the room. As mentioned above, the abnormality condition is satisfied when there is a person other than the care-recipient 500 in the room. The action detection system 300 suppresses the notification by stopping the state transition.

In step (3), the care-giver 501 assists the care-recipient 500 to wake-up from the bed 520. Even at this time, because there are more than one person in the room, the abnormality condition is continuously satisfied.

In step (4), the care-giver 501 assists the care-recipient 500 to sit on the sofa. Even at this time, because there are more than one person in the room, the abnormality condition is continuously satisfied.

In step (5), it is assumed that the care-giver 501 has left the room. Since only care-recipient 500 is present in the room, the abnormality condition is eliminated. Thus, the normality condition is satisfied. As a result, the action detection system 300 restarts state transition and cancels suppression of notification.

As shown in Fig. 10, the action state of care-recipient 500 has changed between before the state transition is stopped and after the state transition resumes. Therefore, if the action state before the stop of the state transition is used as the current action 224 (see Fig. 2) after resuming of the state transition, the action detection unit 255 (see Fig. 2) may make false detection on the action of the care-recipient 500. Therefore, the action detection unit 255 detects the action of the care-recipient 500 when the state transition is resumed, and rewrites the current action 224 with the detected action.

As shown in Fig. 11, when the action state B is detected, the abnormality condition is satisfied. As a result, the transition from the action state A to the action state B is stopped. After that, since the abnormality condition is no longer satisfied, the action detection system 300 resumes the state transition. At this time, the action detection unit 255 detects the action of the care-recipient 500 and resumes the state transition from the detected action. That is, the state transition may be resumed from the action state A, the state transition may be resumed from the action state B, or the state transition may be resumed from another action state C.

As a result, even if the position and the posture of the care-recipient are different between the stop and the resume of the state transition, the action detection system 300 can accurately detect the action of the care-recipient after the state transition is resumed.

### [Control structure of action detection system 300]

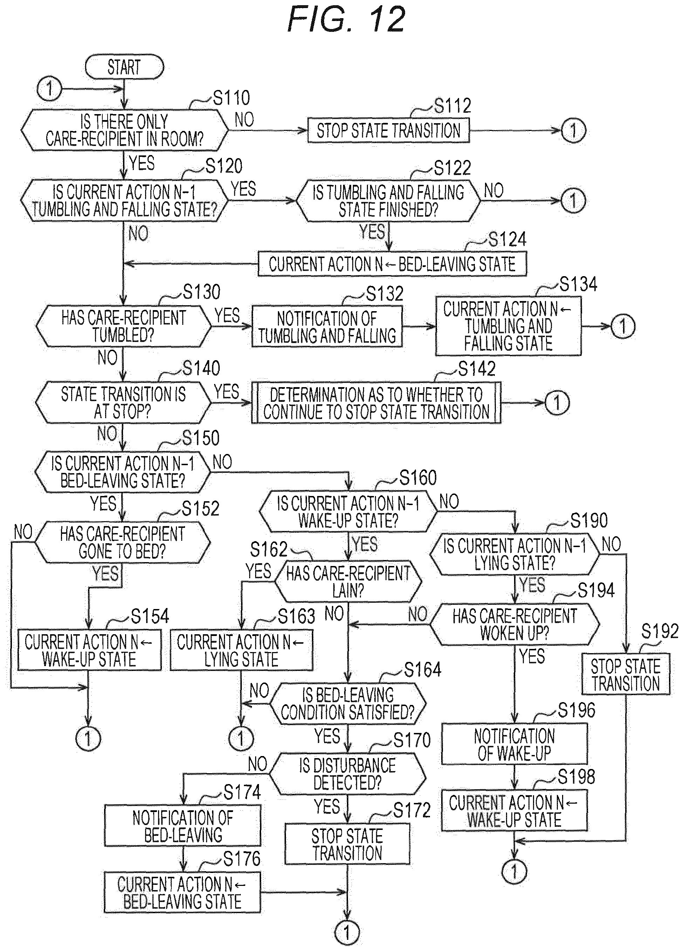

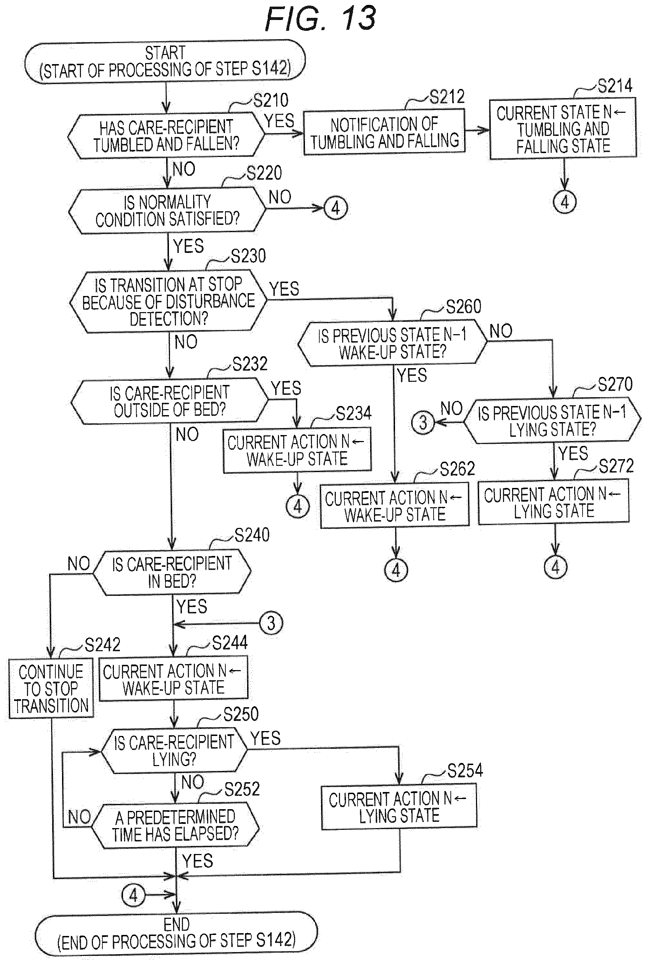

The control structure of the action detection system 300 will be explained with reference to Fig. 12 and Fig. 13. Fig. 12 is a flowchart showing processing of the action detection system 300 when the normality condition is satisfied. Fig. 13 is a flowchart showing processing of the action detection system 300 when the invalid condition is satisfied. The processing in Fig. 12 and Fig. 13 is realized by causing the CPU 102 (see Fig. 14) of the action detection system 300 to execute the program. In other aspects, some or all of the processing may be performed by circuit elements or other hardware.

In step S110, the CPU 102 determines whether or not only a care-recipient is present in the room as the abnormality determination unit 280 (see Fig. 2). When it is determined that only a care-recipient is present in the room (YES in step S110), the CPU 102 determines that the normality condition is satisfied and switches the control to step S120. Otherwise (NO in step S110), the CPU 102 determines that the invalid condition is satisfied, and switches the control to step S112.

In step S112, the CPU 102 stops the state transition in the state transition table 222 (see Fig. 3) as the update unit 285 (see Fig. 2).

In step S120, the CPU 102 determines whether or not the current action 224 of the care-recipient in the image of the (N-1)th frame is the tumbling and falling state. When the CPU 102 determines that the current action 224 of the care-recipient is the tumbling and falling state (YES in step S120), the CPU 102 switches the control to step S122. Otherwise (NO in step S120), the CPU 102 switches the control to step S130.

In step S122, the CPU 102 determines whether or not the tumbling and falling state has ended. When the CPU 102 determines that the tumbling and falling state has ended (YES in step S122), the CPU 102 switches the control to step S124. Otherwise (NO in step S122), the CPU 102 returns the control to step S110.

In step S124, the CPU 102 rewrites the current action 224 of the care-recipient in the image of the N-th frame to the bed-leaving state as the transition control unit 260 (see Fig. 2).

In step S130, the CPU 102 determines whether or not the care-recipient has tumbled as an action detection unit 255 (see Fig. 2). If the CPU 102 determines that the care-recipient has tumbled (YES in step S130), the CPU 102 switches the control to step S132. Otherwise (NO in step S130), the CPU 102 switches the control to step S140.

In step S132, the CPU 102 notifies the mobile terminal 200 of the care-giver (see Fig. 1) that the care-recipient is tumbling and falling as the notification unit 270 (see Fig. 2).

In step S134, the CPU 102 rewrites the current action 224 of the care-recipient in the image of the N-th frame to the tumbling and falling state as the transition control unit 260.

In step S140, the CPU 102 determines, as the transition control unit 260, whether or not the state transition in the state transition table 222 is stopped. When the CPU 102 determines that the state transition in the state transition table 222 is stopped (YES in step S140), the CPU 102 switches the control to step S 142. Otherwise (NO in step S140), the CPU 102 switches the control to step S150.

In step S142, the CPU 102 determines whether or not to continue to stop the state transition in the state transition table 222. This determination is made on the basis of at least one of the following pieces of information (a) to (e), for example.
(a) Type of previous action
(b) Type of current action of care-recipient
(c) Whether or not disturbance has occurred in image
(d) Care-recipient action detection result determined from sensors other than camera
(e) Input to action detection system 300 (e.g., input of administrator, output of other action detection system, or the like)

Whether to continue to stop the state transition, return the current action 224 to the action state before the state transition is stopped, or change the current action 224 to an action state different from the action state before the state transition is stopped is determined with determination processing in step S142. After that, the processing in Fig. 12 is executed again. The detail processing of step S142 will be described later (see Fig. 13).

In step S150, the CPU 102 determines whether or not the current action 224 of the care-recipient in the image of the (N-1)th frame indicates the bed-leaving state. If the CPU 102 determines that the current action 224 of the care-recipient indicates the bed-leaving state (YES in step S150), the CPU 102 switches the control to step S152. Otherwise (NO in step S150), the CPU 102 switches the control to step S160.

In step S152, the CPU 102 determines whether or not the care-recipient has gone to bed. When the CPU 102 determines that the care-recipient has gone to bed (YES in step S152), the CPU 102 switches the control to step S154. Otherwise (NO in step S152), the CPU 102 returns the control to step S110.

In step S154, the CPU 102 rewrites the current action 224 of the care-recipient in the image of the N-th frame as the transition control unit 260 to the wake-up state.

In step S160, the CPU 102 determines whether or not the current action 224 of the care-recipient in the image of the (N-1)th frame is the wake-up state. When the CPU 102 determines that the current action 224 of the care-recipient in the image of the (N-1)th frame is the wake-up state (YES in step S160), the CPU 102 switches the control to step S162. Otherwise (NO in step S160), the CPU 102 switches the control to step S190.

In step S162, the CPU 102 determines whether or not the care-recipient has lain. When the CPU 102 determines that the care-recipient has lain (YES in step S 162), the CPU 102 switches the control to step S163. Otherwise (NO in step S162), the CPU 102 switches the control to step S164.

In step S163, the CPU 102 rewrites the current action 224 of the care-recipient in the image of the N-th frame to the lying state as the transition control unit 260.

In step S164, the CPU 102 determines whether or not the bed-leaving condition is satisfied. As an example, the bed-leaving condition is satisfied when the position of the care-recipient in the image is outside the preset bed region. When the CPU 102 determines that the bed-leaving condition is satisfied (YES in step S164), the CPU 102 switches the control to step S170. Otherwise (NO in step S164), the CPU 102 returns the control to step S110.

In step S170, the CPU 102 determines whether or not disturbance has been detected within the image. When the CPU 102 determines that a disturbance has been detected within the image (YES in step S170), the CPU 102 switches the control to step S172. Otherwise (NO in step S170), the CPU 102 switches the control to step S174.

In step S172, the CPU 102 stops the state transition in the state transition table 222 as the transition control unit 260. That is, when the disturbance is detected within the image, the CPU 102 stops the state transition.

In step S174, the CPU 102 notifies the mobile terminal 200 of the care-giver that the care-recipient is leaving the bed as the notification unit 270. Only when the care-recipient leaves the bed without the influence of the disturbance, the state transition transitions to the bed-leaving state to provide notification of the bed-leaving of the care-recipient.

In step S176, the CPU 102 rewrites the current action 224 of the care-recipient in the image of the N-th frame to the bed-leaving state as the transition control unit 260.

In step S190, the CPU 102 determines whether or not the current action 224 of the care-recipient in the image of the (N-1)th frame is the lying state. When the CPU 102 determines that the current action 224 of the care-recipient in the image of the (N-1)th frame is the lying state (YES in step S190), the CPU 102 switches the control to step S194. Otherwise (NO in step S190), the CPU 102 switches the control to step S192.

In step S192, the CPU 102 stops the state transition in the state transition table 222 as the transition control unit 260.

In step S194, the CPU 102 determines whether or not the care-recipient has woken up. When the CPU 102 determines that the care-recipient has woken up (YES in step S194), the CPU 102 switches the control to step S196. Otherwise (NO in step S194), the CPU 102 switches the control to step S164.

In step S196, the CPU 102 notifies the mobile terminal 200 of the care-giver that the care-recipient has woken up as the notification unit 270.

In step S198, the CPU 102 rewrites the current action 224 of the care-recipient in the image of the N-th frame to the wake-up state as the transition control unit 260.

Next, processing of action detection system 300 that is performed when invalid condition is satisfied will be explained with reference to Fig. 13.

In step S210, the CPU 102 determines whether or not the care-recipient has tumbled and fallen. When the CPU 102 determines that care-recipient has tumbled and fallen (YES in step S210), the CPU 102 switches the control to step S212. Otherwise (NO in step S210), the CPU 102 switches the control to step S220.

In step S212, the CPU 102 notifies the mobile terminal 200 of the care-giver that the care-recipient has tumbled and fallen as the notification unit 270.

In step S214, the CPU 102 rewrites the current action 224 of the care-recipient in the image of the N-th frame to the tumbling and falling state as the transition control unit 260.

In step S220, the CPU 102 determines whether or not the normality condition is satisfied. When the CPU 102 determines that the normality condition is satisfied (YES in step S220), the CPU 102 switches the control to step S230. Otherwise (NO in step S220), the CPU 102 ends the action determination processing shown in Fig. 13.

An example of a case where the normality condition is resolved includes a case where the reset state of the action detection system 300 is resolved. In this case, there is a possibility that the current action 224 has also been reset, and the next action of the care-recipient cannot be detected. Therefore, when the reset of the action detection system 300 is canceled and the normality condition is satisfied, the CPU 102 detects the action of the care-recipient, sets the action to the current action 224, and then resumes processing of Fig. 12.

As the detection method of the current action 224 at restarting processing, the CPU 102 detects the bed-leaving state of the care-recipient when the position of the care-recipient in the image is outside the bed. Note that the CPU 102 may detect the action state of the care-recipient by using a distance sensor instead of the camera. In one aspect, the distance sensor is installed in the bed and detects the distance from the bed to the surrounding objects. The CPU 102 detects the care-recipient when the distance changes by a predetermined value or more. In another aspect, a distance sensor is provided on a wall surface of the bed. The CPU 102 may detect the thickness of the person on the bed based on the detection result by the distance sensor and determine whether the care-recipient is lying or has woken up based on the thickness.

The CPU 102 may detect the action state of the care-recipient using a mat-like gravity sensor instead of the camera. More specifically, the gravity sensor is provided so as to straddle the bed region and its peripheral region. In the case where the gravity is detected both in the bed region and outside the bed region, the CPU 102 detects the wake-up of the care-recipient. When the gravity is detected only within the bed region, the CPU 102 detects the lying state of the care-recipient.

In step S230, the CPU 102 determines whether or not the state transition in the state transition table 222 is stopped due to disturbance. When the CPU 102 determines that the state transition in the state transition table 222 is stopped due to disturbance (YES in step S230), the CPU 102 switches the control to step S260. Otherwise (NO in step S230), the CPU 102 switches the control to step S232.

In step S232, the CPU 102 determines whether or not the position of the care-recipient in the image is outside the bed. When the CPU 102 determines that the position of care-recipient in the image is outside the bed (YES in step S232), the CPU 102 switches the control to step S234. Otherwise (NO in step S232), the CPU 102 switches the control to step S240.

In step S234, the CPU 102 rewrites the current action 224 of the care-recipient in the image of the N-th frame to the wake-up state as the transition control unit 260.

In step S240, the CPU 102 determines whether or not the position of the care-recipient in the image is within the bed. When the CPU 102 determines that the position of the care-recipient in the image is within the bed (YES in step S240), the CPU 102 switches the control to step S244. Otherwise (NO in step S240), the CPU 102 switches the control to step S242.

In step S242, the CPU 102 continues to stop the state transition in the state transition table 222. That is, the CPU 102 executes the action determination processing shown in Fig. 13 again.

In step S244, the CPU 102 rewrites the current action 224 of the care-recipient in the image of the N-th frame to the wake-up state as the transition control unit 260.

In step S250, the CPU 102 determines whether the care-recipient is lying or not. When the CPU 102 determines that care-recipient is lying (YES in step S250), the CPU 102 switches the control to step S254. Otherwise (NO in step S250), the CPU 102 switches the control to step S252.

In step S252, the CPU 102 determines whether or not a predetermined time has elapsed since the processing of step S250 was executed. When the CPU 102 determines that a certain period of time has elapsed since the processing of step S250 was executed (YES in step S252), the CPU 102 executes the action determination processing shown in Fig. 13 again. Otherwise (NO in step S252), the CPU 102 executes the processing of step S250 again.

In step S254, the CPU 102 rewrites the current action 224 of the care-recipient in the image of the N-th frame to the lying state as the transition control unit 260.

In step S260, the CPU 102 determines whether or not the previous action of the care-recipient in the (N-1)th frame is a wake-up state. When the CPU 102 determines that the previous action of the care-recipient in the (N-1)th frame is the wake-up state (YES in step S260), the CPU 102 switches the control to step S262. Otherwise (NO in step S260), the CPU 102 switches the control to step S270.

In step S262, the CPU 102 rewrites the current action 224 of the care-recipient in the image of the N-th frame to the wake-up state as the transition control unit 260.

In step S270, the CPU 102 determines whether or not the previous action of the care-recipient in the (N-1)th frame is the lying state. When the CPU 102 determines that the previous action of the care-recipient in the (N-1)th frame is the lying state (YES in step S270), the CPU 102 switches the control to step S272. Otherwise (NO in step S270), the CPU 102 switches the control to step S244.

In step S272, the CPU 102 rewrites the current action 224 of the care-recipient in the image of the N-th frame to the lying state as the transition control unit 260.

### [Hardware configuration of action detection device 100]

An example of the hardware configuration of the action detection device 100 shown in Fig. 1 will be described with reference to Fig. 14. Fig. 14 is a block diagram showing a main hardware configuration of the action detection device 100. As illustrated in Fig. 14, the action detection device 100 includes a ROM (Read Only Memory) 101, a CPU 102, a RAM (Random Access Memory) 103, a network interface 104, a camera interface 105, and a storage device 120.

The ROM 101 stores an operating system, an action detection program 122 according to the present embodiment, and the like. The CPU 102 controls the operation of the action detection device 100 by executing various programs such as the operating system and the action detection program 122. The RAM 103 functions as a working memory, and temporarily stores various data necessary for executing the action detection program 122.

A communication device such as an antenna or a NIC (Network Interface Card) is connected to the network interface 104. The action detection device 100 transmits and receives data to and from other communication terminals via the communication device. For example, the other communication terminals include a camera 50 (see Fig. 1), a mobile terminal 200 (see Fig. 1), a server, other terminals, and the like. The action detection device 100 may be configured so that the action detection program 122 can be downloaded from the server via the network.

The camera interface 105 is an interface for connecting the camera 50 and the action detection device 100 in a wired or wireless manner. The action detection device 100 acquires image from the camera 50 via the camera interface 105. The camera 50 includes, for example, a network camera or other image-capturing device capable of shooting a subject. The camera 50 may be integrally configured with the action detection device 100 or may be configured separately from the action detection device 100 as shown in Fig. 14.

The storage device 120 is, for example, a storage medium such as a hard disk or an external storage device. As an example, the storage device 120 stores an action detection program 122, a state transition table 222 (see Fig. 3), a current action 224 (see Fig. 2), a notification action information 226 (see Fig. 8), and the like.

It should be noted that the action detection program 122 may be provided as a part of an arbitrary program, not as a single program. In this case, the processing in accordance with the present embodiment is realized in cooperation with an arbitrary program. Programs that do not include some of such modules do not depart from the spirit of the action detection device 100 according to the present embodiment. Furthermore, some or all of the functions provided by the action detection program 122 according to the present embodiment may be implemented by dedicated hardware. In addition, the action detection device 100 and the mobile terminal 200 may cooperate to realize some or all of the functions provided by the action detection program 122. In addition, the action detection device 100 may be configured in a form like so-called cloud service where at least one server implements processing according to the present embodiment.

### [Brief Summary]

As described above, the action detection system 300 according to the present embodiment reduces the type of the notification target action and updates the transition relationship of the state transition table 222, when a predetermined invalid condition indicating that it is not suitable to determine the type of the action of the care-recipient is satisfied. As a result, false notification occurring in an unintended situation is suppressed, and the burden on care-giver is reduced.

### <Second embodiment>

The action detection system 300 according to the second embodiment can determine more types of actions than the action detection system 300 according to the first embodiment.

The action detection system 300 according to the second embodiment will be described with reference to Fig. 15 to Fig. 17. Fig. 15 shows content of the state transition table 222 according to the second embodiment.

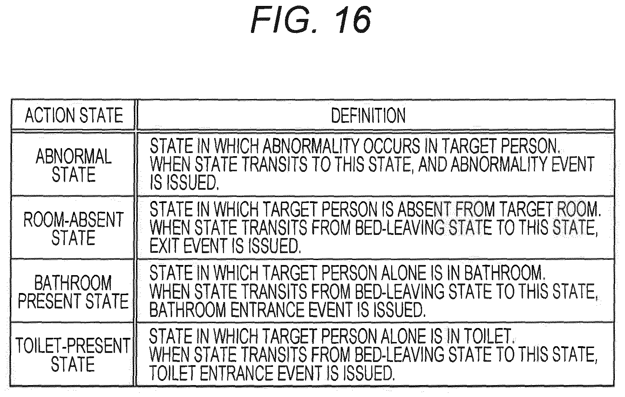

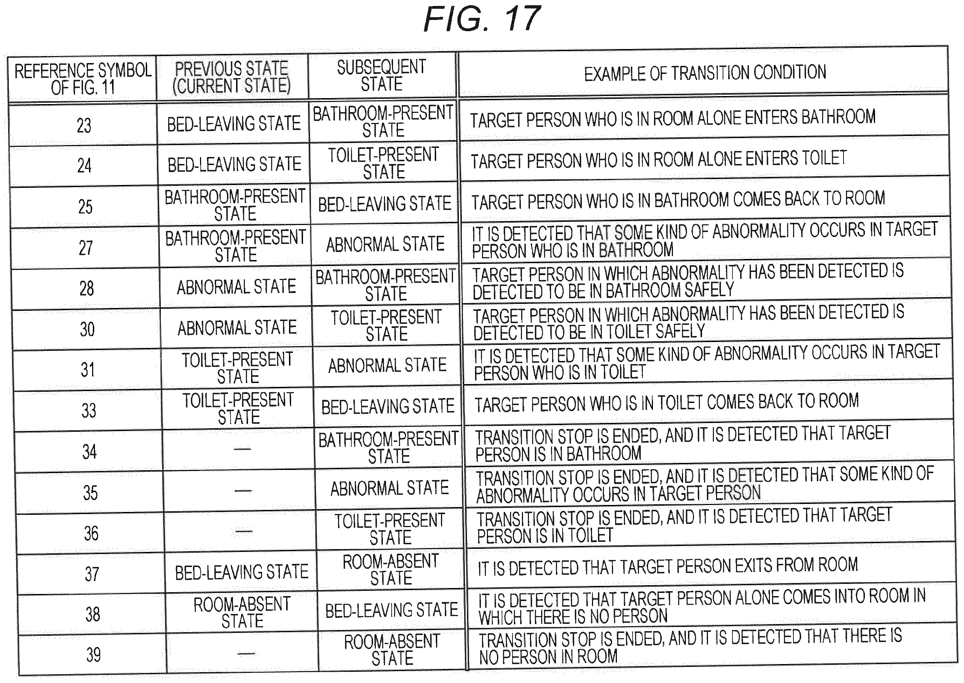

Fig. 16 shows the definition of each action state prescribed in the state transition table 222 according to the second embodiment. Fig. 17 shows transition condition from each action state prescribed in the state transition table 222 according to the second embodiment.

As shown in Fig. 15, in the state transition table 222, a transition relationship between actions of plural types is defined. In the example of Fig. 15, in the state transition table 222, a bed-leaving state 222A, a room-absent state 222E, a bathroom-present state 222F, an abnormal state 222G, and a toilet-present state 222H are defined as the action state. The definition of these action states is shown in Fig. 16.

As shown in Fig. 15, the action state can transition from the bed-leaving state 222A to the room-absent state 222E, the bathroom-present state 222F, or the toilet-present state 222H. From the room-absent state 222E, the action state can transition to the bed-leaving state 222A. From the bathroom-present state 222F, the action state can transition to the bed-leaving state 222A or the abnormal state 222G. From the abnormal state 222G, the action state can transition to the bathroom-present state 222F or the toilet-present state 222H. From the toilet-present state 222H, the action state can transition to bed-leaving state 222A or abnormal state 222G.

Based on the state transition table 222, the action detection unit 255 (see Fig. 2) specifies the type of action to which transition can be made from the current action 224 (see Fig. 2), and detects the specified transitionable action by using the input image.

A more specific action detection method will be described. The bed region, indoor region, bath region, and toilet region are set in advance in the image. The bed region, the indoor region, the bath region, and the toilet region may be set in advance manually or may be automatically detected by image processing techniques such as template matching.

When the position of the care-recipient in the image is not included in the indoor region, the action detection unit 255 detects the room-absent state of the care-recipient. Alternatively, when the care-recipient in the image passes through the entrance of the room set in advance, the action detection unit 255 detects the room-absent state of the care-recipient.

The action detection unit 255 detects the bathroom-present state 222F, the abnormal state 222G, or the toilet-present state 222H according to the use condition of the bathroom or the toilet. As an example, when the length of time for which the position of the care-recipient in the image is in the bathroom region is shorter than the certain period of time, the action detection unit 255 detects the bathroom-present state 222F. When the length of time for which the position of the care-recipient in the image is in the bathroom region is longer than the certain period of time, the action detection unit 255 detects the abnormal state 222G of the care-recipient. When the length of time for which the position of the care-recipient in the image is in the toilet region is shorter than the certain period of time, the action detection unit 255 detects the toilet-present state 222H. When the length of time for which the position of care-recipient in image is in toilet region is longer than the certain period of time, the action detection unit 255 detects the abnormal state 222G of the care-recipient.

Fig. 17 shows transition condition for transition from each action state prescribed in the state transition table 222 to another action state. More specifically, the transition condition 23 indicates the transition condition from the bed-leaving state 222A to the bathroom-present state 222F. The transition condition 24 indicates a transition condition from the bed-leaving state 222A to the toilet-present state 222H. The transition condition 25 indicates a transition condition from the bathroom-present state 222F to the bed-leaving state 222A. The transition condition 27 indicates a transition condition from the bathroom-present state 222F to the abnormal state 222G. The transition condition 28 indicates a transition condition from the abnormal state 222G to the bathroom-present state 222F. The transition condition 30 indicates a transition condition from the abnormal state 222G to the toilet-present state 222H. The transition condition 31 indicates a transition condition from the toilet-present state 222H to the abnormal state 222G. The transition condition 33 indicates a transition condition from the toilet-present state 222H to the bed-leaving state 222A. The transition condition 34 indicates a transition condition from the initial state where the action state is not determined to the bathroom-present state 222F. The transition condition 35 indicates a transition condition from the initial state where the action state is not determined to the abnormal state 222G. The transition condition 36 indicates a transition condition from the initial state where the action state is not determined to the toilet-present state 222H. The transition condition 37 indicates a transition condition from the bed-leaving state 222A to the room-absent state 222E. The transition condition 38 indicates a transition condition from the room-absent state 222E to the bed-leaving state 222A. The transition condition 39 indicates a transition condition from the initial state where the action state is not determined to the room-absent state 222E.

For the transition condition shown in Fig. 17, other conditions may be specified. For example, the transition conditions 27 and 31 may be satisfied when the bathroom-present state or the toilet-present state of the care-recipient is extremely longer than usual. Alternatively, the transition conditions 27 and 31 may be satisfied when bathroom or toilet water is continuously used for a prolonged period of time. Whether the transition conditions 23 and 24 are satisfied or not may be determined based on the usage of water or toilet. Whether the transition conditions 37 and 38 are satisfied or not may be determined based on the opening and closing operation of the indoor door. The transition conditions shown in Fig. 17 can be set by the administrator of the action detection system 300.

### [Brief Summary]

As described above, the action detection system 300 according to the second embodiment can detect more types of actions than the first embodiment. As a result, the care-giver can accurately find the action of the care-recipient.

### <Summary>

An action detection system includes an acquisition unit for acquiring operation data indicating an operation of a subject, a storage unit for storing a state transition table for specifying transition relationship between a plurality of types of actions, a type of a notification target action, and a type of a current action of the subject, an action detection unit for specifying a transitionable action to which transition can be made from the current action on the basis of the state transition table, and detecting the transitionable action on the basis of the operation data, a transition control unit for updating the current action to the new action in a case where the action detection unit detects the new action, a notification unit for, in a case where the new action is detected and the new action is the notification target action, providing notification of a type of the new action, and an update unit for, in a case where a first condition defined in advance indicating that it is not suitable to determine the type of the action of the subject is satisfied, reducing the type of the notification target action and updating the transition relationship of the state transition table.

Preferably, while the first condition is satisfied, the type of transitionable action is specified on the basis of the updated state transition table.

Preferably, the first condition is satisfied, the update unit makes the type of the notification target action be zero.

Preferably, in a case where a second condition defined in advance indicating that it is suitable to determine the type of the action of the subject is satisfied, the update unit returns the updated state transition table back to the pre-updated state transition table.

### Preferably, the operation data is an image.

Preferably, the first condition is satisfied in at least one of: a case where disturbance occurs in the operation data; a case where a person other than the subject is included in the operation data; a case where the action detection system is initialized; and an error occurs in the action detection system.

Preferably, the type of the notification target action includes at least one of wake-up of the subject, bed-leaving of the subject, fall of the subject, tumbling of the subject, going-to-bed of the subject, lying of the subject, room-entering of the subject, and room-exiting of the subject.

The embodiments disclosed this time should be considered as examples in all respects and not restrictive. The scope of the present invention is defined not by the description above but by the claims, meaning equivalent to the claims and all changes within the scope are intended to be included.

### Reference Signs List

6, 7, 8, 10, 11, 12, 13, 15, 16, 17, 18, 20, 21, 22, 23, 24, 25, 27, 28, 30, 31, 33, 34, 35, 36, 37, 38, 39 transition condition, 50 camera, 100 action detection device, 101 ROM, 102 CPU, 103 RAM, 104 network interface, 105 camera interface, 120 storage device, 122 action detection program, 150 acquisition unit, 200 mobile terminal, 222 state transition table, 222A bed-leaving state, 222B wake-up state, 222C lying state, 222D tumbling and falling state, 222E room-absent state, 222F bathroom-present state, 222G abnormal state, 222H toilet-present state, 224 current action, 226 notification action information, 250 moving object detection unit, 252 person determination unit, 255 action detection unit, 260 transition control unit, 270 notification unit, 280 abnormality determination unit, 285 update unit, 290 normality determination unit, 300 action detection system, 500 care-recipient, 501 care-giver, 503, 504 person, 505 curtain, 520 bed.

## Claims

1. An action detection system comprising:
an acquisition unit for acquiring operation data indicating an operation of a subject;
a storage unit for storing a state transition table for specifying transition relationship between a plurality of types of actions, a type of a notification target action, and a type of a current action of the subject;
an action detection unit for specifying a transitionable action to which transition can be made from the current action on the basis of the state transition table, and detecting the transitionable action on the basis of the operation data;
a transition control unit for updating the current action to the new action in a case where the action detection unit detects the new action;
a notification unit for, in a case where the new action is detected and the new action is the notification target action, providing notification of a type of the new action; and
an update unit for, in a case where a first condition defined in advance indicating that it is not suitable to determine the type of the action of the subject is satisfied, reducing the type of the notification target action and updating the transition relationship of the state transition table.

2. The action detection system according to claim 1, wherein while the first condition is satisfied, the type of transitionable action is specified on the basis of the updated state transition table.

3. The action detection system according to claim 1 or 2, wherein in a case where the first condition is satisfied, the update unit makes the type of the notification target action be zero.

4. The action detection system according to any one of claims 1 to 3, wherein in a case where a second condition defined in advance indicating that it is suitable to determine the type of the action of the subject is satisfied, the update unit returns the updated state transition table back to the pre-updated state transition table.

5. The action detection system according to any one of claims 1 to 4, wherein the operation data is an image.

6. The action detection system according to any one of claims 1 to 5, wherein the first condition is satisfied in at least one of: a case where disturbance occurs in the operation data; a case where a person other than the subject is included in the operation data; a case where the action detection system is initialized; and an error occurs in the action detection system.

7. The action detection system according to any one of claims 1 to 6, wherein the type of the notification target action includes at least one of wake-up of the subject, bed-leaving of the subject, fall of the subject, tumbling of the subject, going-to-bed of the subject, lying of the subject, room-entering of the subject, and room-exiting of the subject.

8. An action detection system comprising:
an acquisition unit for acquiring operation data indicating an operation of a subject;
a storage unit for storing a state transition table for specifying transition relationship between a plurality of types of actions, and a type of a current action of the subject;
an action detection unit for specifying a transitionable action to which transition can be made from the current action on the basis of the state transition table, and detecting the transitionable action on the basis of the operation data;
a transition control unit for updating the current action to a new action in a case where the action detection unit detects the new action; and
a notification unit for, in a case where the new action is detected and the new action is a notification target action defined in advance, providing notification of a type of the new action,
wherein in a case where a condition defined in advance indicating that it is not suitable to determine the type of the action of the subject is satisfied, the notification unit stops providing notification of the notification target action defined in advance.

9. An action detection device comprising:
an acquisition unit for acquiring operation data indicating an operation of a subject;
a storage unit for storing a state transition table for specifying transition relationship between a plurality of types of actions, a type of a notification target action, and a type of a current action of the subject;
an action detection unit for specifying a transitionable action to which transition can be made from the current action on the basis of the state transition table, and detecting the transitionable action on the basis of the operation data;
a transition control unit for updating the current action to a new action in a case where the action detection unit detects the new action;
a notification unit for, in a case where the new action is detected and the new action is the notification target action, providing notification of a type of the new action; and
an update unit for, in a case where a first condition defined in advance indicating that it is not suitable to determine the type of the action of the subject is satisfied, reducing the type of the notification target action and updating the transition relationship of the state transition table.

10. An action detection method comprising:
a step of acquiring operation data indicating an operation of a subject;
a step of preparing a state transition table for specifying transition relationship between a plurality of types of actions, a type of a notification target action, and a type of a current action of the subject;
a step of specifying a transitionable action to which transition can be made from the current action on the basis of the state transition table, and detecting the transitionable action on the basis of the operation data;
a step of updating the current action to a new action in a case where the new action is detected in the detecting step;
a step of, in a case where the new action is detected and the new action is the notification target action, providing notification of a type of the new action; and
a step of, in a case where a first condition defined in advance indicating that it is not suitable to determine the type of the action of the subject is satisfied, reducing the type of the notification target action and updating the transition relationship of the state transition table.

11. An action detection program causing a computer to execute:
a step of acquiring operation data indicating an operation of a subject;
a step of preparing a state transition table for specifying transition relationship between a plurality of types of actions, a type of a notification target action, and a type of a current action of the subject;
a step of specifying a transitionable action to which transition can be made from the current action on the basis of the state transition table, and detecting the transitionable action on the basis of the operation data;
a step of updating the current action to a new action in a case where the new action is detected in the detecting step;
a step of, in a case where the new action is detected and the new action is the notification target action, providing notification of a type of the new action; and
a step of, in a case where a first condition defined in advance indicating that it is not suitable to determine the type of the action of the subject is satisfied, reducing the type of the notification target action and updating the transition relationship of the state transition table.
